# EUROPEAN PATENT SPECIFICATION

(11) **EP 2 250 887 B1**
(45) Date of publication and mention of the grant of the patent: **24.04.2013**
(21) Application number: 10174817.6
(22) Date of filing: 27.03.2003
(51) Int. Cl.: A01N 37/18, A61K 45/06, A61K 31/165, A61K 39/02, A61K 31/65, A61P 39/04, A61P 31/04

(54) **Use of non-antibacterial tetracycline analog CMT-1002 and formulations thereof for the treatment of bacterial exotoxins**
Verwendung des nicht-antibakteriellen Tetrazyklin-analogs CMT-1002, und Zubereitungen daraus, zur Behandlung von bakteriellen Exotoxinen
Utilisation de l'analogue de tétracycline anti-bactérien CMT-1002, et des formulations de celui-ci, pour le traitement des exotoxines bactériens

(30) Priority: 29.03.2002 US 368478 P
(43) Date of publication of application: 17.11.2010
(62) Divisional of application: 03745650.6
(73) Proprietor: The Research Foundation Of The State University Of New York, Albany NY 12201 (US)
(72) Inventor: Golub, Lorne M., Smithdown, NY 11787 (US); Walker, Stephen G., East Setauket, NY 11733-2605 (US)
(74) Representative: Jansen, Cornelis Marinus

(56) References cited:
- EP-A2- 0 736 302
- WO-A1-00/28983
- WO-A1-01/87823
- US-A- 4 704 383
- US-A- 5 308 839
- US-A- 5 919 775
- CRAIG R G ET AL: "A chemically modified tetracycline inhibits streptozotocin-induced diabetic depression of skin collagen synthesis and steady-state type I procollagen mRNA" BIOCHIMICA ET BIOPHYSICA ACTA. MOLECULAR CELL RESEARCH, ELSEVIER SCIENCE PUBLISHERS, AMSTERDAM, NL LNKD- DOI:10.1016/S0167-4889(98)00008-1, vol. 1402, no. 3, 24 April 1998 (1998-04-24), pages 250-260, XP004277775 ISSN: 0167-4889
- LONN U ET AL: "Cytotoxicity, calmodulin and DNA lesions in cells treated with streptozotocin" BIOCHEMICAL PHARMACOLOGY, PERGAMON, OXFORD, GB LNKD- DOI:10.1016/0006-2952(88)90694-6, vol. 37, no. 18, 15 September 1988 (1988-09-15), pages 3441-3446, XP023758171 ISSN: 0006-2952 [retrieved on 1988-09-15]
- DRUM C L ET AL: "Structural basis for the activation of anthrax adenylyl cyclase exotoxin by calmodulin" 24 January 2002 (2002-01-24), NATURE 20020124 GB LNKD- DOI:10.1038/415396A, VOL. 415, NR. 6870, PAGE(S) 396 - 402 , XP002604982 ISSN: 0028-0836 * page 396 *
- KOCER S S ET AL: "Metalloproteinase inhibitors, nonantimicrobial chemically modified tetracyclines, and Ilomastat block Bacillus anthracis lethal factor activity in viable cells" INFECTION AND IMMUNITY 200511 US LNKD- DOI:10.1128/IAI.73.11.7548-7557.2005, vol. 73, no. 11, November 2005 (2005-11), pages 7548-7557, XP002604848 ISSN: 0019-9567

## Description

### BACKGROUND OF THE INVENTION

When bacteria attack a host, there is an incubation period during which there are mild or no symptoms. The incubation period varies among bacteria.

Once inside the host, some bacteria begin producing exotoxins. These exotoxins damage host tissue and organs, sometimes causing a sudden onset of hyperacute illness which progresses to shock, coma and death.

For example, an inhalation infection with *Bacillus anthracis* (anthrax) can have an incubation period of 3 to 60 days. Death from anthrax inhalation is considered inevitable if untreated, and probable in as many as 95% of treated cases if therapy is begun more than 48 hours after the onset of symptoms.

The lack of warning symptoms, sudden onset and almost absolute mortality, among other factors, have made anthrax a choice disease for use as a biological weapon of mass destruction. The threat of such a weapon has heightened research into modes of treatment and prevention of anthrax.

Of particular interest is a treatment for individuals at high risk of exposure, as well as for those who may have been exposed to anthrax, but are without symptoms. Currently, antibiotics such as ciprofloxacin are prescribed for such individuals. Due to the variably long incubation period with the inhaled form of anthrax, individuals potentially exposed are often put on an antibiotic therapy for sixty days.

Antibiotics target the bacteria itself. Often, in bacterial infections such as anthrax, the conventional therapy of antibiotics is administered too late. For example, ciprofloxacin has substantially no effect on the exotoxins released by the bacteria, which is the eventual cause of death. Therefore, once the infection has progressed to the point where sufficient exotoxin has been released, antibiotics alone have little or no effect. Even if the bacteria have been eliminated, remaining exotoxins may continue to cause tissue damage leading to death.

The problem with prescribing antibiotics as a treatment for individuals who may or may not be infected with bacteria such as anthrax is antibiotic resistance. The Center for Disease Control (CDC) has called antibiotic resistance one of the world's most pressing health problems. See, www.cdc.gov/antibioticresistance/. Thus, prescribing a sixty day course of antibiotics to potentially anthrax-exposed individuals increases the likelihood of antibiotic-resistant bacterial strains.

Hence, the prior art treatments for exotoxin-releasing bacterial infections, such as anthrax, are disadvantageous. Current treatments, such as administration of ciprofloxacin, do not target the deadly exotoxins or protect against tissue and organ damage. Moreover, a potentially unnecessary sixty day course of antibiotics may lead to antibiotic resistant bacterial strains.

An ideal treatment for exotoxin-releasing bacteria would be the targeting and neutralization, or disabling of the deadly exotoxins. Such treatment would provide protection against the exotoxins without using antibiotics until actual bacterial infection has been confirmed.

The compound, tetracycline is a member of a class of antibiotic compounds that is referred to as the tetracyclines, tetracycline compounds, tetracycline derivatives and the like. The compound tetracycline exhibits the following general structure:

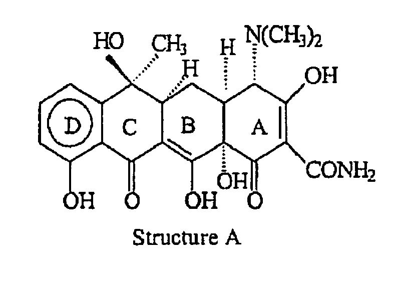

The numbering system of the tetracycline ring nucleus is as follows:

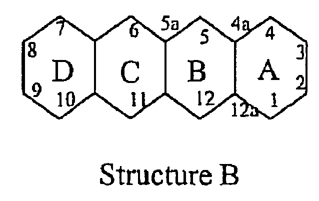

Tetracycline, as well as the terramycin and aureomycin derivatives, exist in nature, and are well known antibiotics. Natural tetracyclines may be modified without losing their antibiotic properties, although certain elements must be retained. The modifications that may and may not be made to the basic tetracycline structure have been reviewed by Mitscher in The Chemistry of Tetracyclines. Chapter 6, Marcel Dekker, Publishers, New York (1978). According to Mitscher, the substituents at positions 5-9 of the tetracycline ring system may be modified without the complete loss of antibiotic properties.

Changes to the basic ring system or replacement of the substituents at positions 4 and 10-12, however, generally lead to synthetic tetracyclines with substantially less or effectively no antimicrobial activity. Some examples of chemically modified non-antibacterial tetracyclines (hereinafter CMTs) are 4-dedimethylaminotetracyline, 4-dedimethylaminosancycline (6-demethyl-6-deoxy-4-dedimethylaminotetracycline), 4-dedimethylaminominocycline (7-dimethylamino-6-demethyl-6-deoxy-4-dedimethylaminotetracycline), and 4-dedimethylaminodoxycycline (5-hydroxy-6-deoxy-4-dedimethyaminotetracycline).

In addition to their antimicrobial properties, tetracyclines have been described as having a number of other uses. For example, tetracyclines are also known to inhibit the activity of collagen destructive enzymes produced by mammalian (including human) cells and tissues by non-antibiotic mechanisms. Such enzymes include the matrix metalloproteinases (MMPs), including collagenases (MMP-1, MMP-8 and MMP-13), gelatinases (MMP-2 and MMP-9), and others (e.g. MMP-12, MMP-14). See Golub et al., J. Periodont. Res. 20:12-23 (1985); Golub et al. Crit. Revs. Oral Biol. Med. 2:297-322 (1991); U.S. Patent Nos. 4,666,897; 4,704,383; 4,935,411; 4,9354,412. Also, tetracyclines have been known to inhibit wasting and protein degradation in mammalian skeletal muscle, U.S. Pat. No. 5,045,538, to inhibit inducible NO synthase, U.S. Patent Nos. 6,043,231 and 5,523,297, and phospholipase A₂, U.S. Patent Nos. 5,789,395 and 5,919,775, and to enhance IL-10 production in mammalian cells. These properties cause the tetracyclines to be useful in treating a number of diseases.

The object of this invention is to provide a method for protecting a mammal infected by, or at risk of exposure to, bacteria that produce exotoxins such as anthrax without the risk of antibiotic resistance.

### SUMMARY OF THE INVENTION

It has now been discovered that these and other objectives can be achieved by the following methods.

In a first embodiment of the invention, a method for protecting a mammal at risk of acquiring a condition associated with bacteria that produce a calmodulin exotoxin, a metalloproteinase exotoxin, or both, is provided.

In a second embodiment, a method for treating a mammal having a condition associated with bacteria that produce a calmodulin exotoxin, a metalloproteinase exotoxin, or both, is provided.

In a third embodiment, a method for protecting a mammal that has received or is scheduled to receive a vaccine against a bacteria that produces a calmodulin exotoxin, metalloproteinase exotoxin, or both, is provided.

In the three embodiments of the invention as claimed listed above, the bacteria is selected from the group consisting of *Bacillus anthracis, Clostridium perfringens, Bordetella pertussis, or Bacteriodes fragilis.*

In one embodiment, the methods comprise administering to the mammal an effective amount of a non-antibacterial tetracycline which is CMT-1002, or a pharmaceutically acceptable salt thereof.

In another embodiment, the method further includes administration of an antibiotic, along with the non-antibacterial tetracycline.

In an additional embodiment the tetracycline is administered before a vaccine is administered, at the same time that a vaccine is administered, or after a vaccine is administered.

### DETAILED DESCRIPTION

The invention relates to treating conditions associated with a bacterial exotoxin with a tetracycline derivative, which is CMT-1002.

Also disclosed are antibacterial tetracycline compounds administered in a non-antibacterial amount. The tetracycline derivative may be any such derivative having clinically significant antibacterial activity. Some examples of antibacterial tetracycline derivatives include tetracycline, as well as the 5-OH (oxytetracycline, e.g. terramycin™) and 7-Cl (chlorotetracycline, e.g., aureomycin™) derivatives, which exist in nature. Semisynthetic tetracyclines include, for example, doxycycline, minocycline and sancycline.

The amount of a tetracycline compound that has substantially no antibacterial activity is an amount that does not significantly prevent the growth of bacteria. For example, tetracycline compounds that have significant antibacterial activity could be administered in an amount which is 10-80% of the antibacterial amount. Also, the antibacterial tetracycline compound could be administered in an amount which is 40-70% of the antibacterial amount.

Some examples of antibacterial amounts of members of the tetracycline family include 100mg/day ofdoxycycline, 200mg/day of minocycline, 250mg of tetracycline four times a day, 1000mg/day of oxytetracycline, 600mg/day of demeclocycline and 600mg/day of lymecycline.

Examples of antibacterial tetracyclines administered in a non-antibacterial amount are depicted in Table 1, as follows:

**Table 1**

| Drug | Maximum Non-Antimicrobial Dose | Plasma Antimicrobial Threshold Level |
|---|---|---|
| Doxycycline | 20 mg b.i.d | 1.0 mcg/mL |
| Minocycline | 38 mg q.d. | 0.8 mcg/mL |
| Tetracycline | 60 mg q.d. | 0.5 mcg/mL |

Doxycycline administered at a 20 milligram dose twice daily is sold for the treatment of periodontal disease by CollaGenex Pharmaceuticals, Inc. of Newtown, Pennsylvania under the trademark Periostat ®.

In the claimed invention, a non-antibacterial tetracycline compound which is CMT-1002 administered. A class of compounds has been defined which are structurally related to the antibiotic tetracyclines, but which have had their antibiotic activity substantially or completely eliminated by chemical modification. Substantial elimination of antibiotic activity occurs when the antibiotic activity is ten times less than that of tetracycline, and preferably five times less than that of doxycycline.

One such group of chemically modified nonantibacterial tetracyclines (CMT's) includes any of the antibacterial 4-dedimethylaminotetracycline derivatives. Some examples of non-antibacterial tetracyclines include those compounds disclosed generically or specifically in co-pending U.S. patent application serial no. 09/573,654 filed on May 18, 2000.

Some examples of 4-dedimethylaminotetracycline derivatives include the following general formulae (I) through (IV):

### General Formula (I)

Structure A represents the 4-dedimethylaminosancycline (CMT-3) derivatives 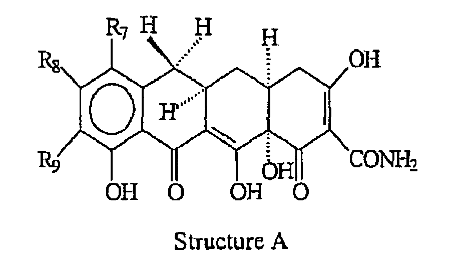 wherein R7, R8, and R9 taken together in each case, have the following meanings:

| | R7 | R8 | R9 |
|---|---|---|---|
| | | | |
| | azido | hydrogen | hydrogen |
| | dimethylamino | hydrogen | azido |
| | hydrogen | hydrogen | azido |
| | dimethylamino | hydrogen | amino |
| | acylamino | hydrogen | hydrogen |
| | amino | hydrogen | nitro |
| | hydrogen | hydrogen | (N,Ndimethyl)glycylamino |
| | amino | hydrogen | amino |
| | hydrogen | hydrogen | ethoxythiocarbonylthio |
| | dimethylamino | hydrogen | acylamino |
| | dimethylamino | hydrogen | diazonium |
| | dimethylamino | chloro | amino |
| | hydrogen | chloro | amino |
| | amino | chloro | amino |
| | acylamino | chloro | acylamino |
| | amino | chloro | hydrogen |
| | acylamino | chloro | hydrogen |
| | monoalkylamino | chloro | amino |
| | nitro | chloro | amino |
| | dimethylamino | chloro | acylamino |
| | dimethylamino | chloro | dimethylamino |
| | acylamino | hydrogen | hydrogen |
| | hydrogen | hydrogen | acylamino |
| (CMT-301) | bromo | hydrogen | hydrogen |
| (CMT-302) | nitro | hydrogen | hydrogen |
| (CMT-303) | hydrogen | hydrogen | nitro |
| (CMT-304) | acetamido | hydrogen | hydrogen |
| (CMT-305) | hydrogen | hydrogen | acetamido |
| (CMT-306) | hydrogen | hydrogen | dimethylamino |
| (CMT-307) | amino | hydrogen | hydrogen |
| (CMT-308) | hydrogen | hydrogen | amino |
| (CMT-309) | hydrogen | hydrogen | dimethylaminoacetamido |
| (CMT-310) | dimethylamino | hydrogen | hydrogen |
| (CMT-311) | hydrogen | hydrogen | galmitamide |

| | R7 | R8 | R9 | R2 |
|---|---|---|---|---|
| (CMT-312) | hydrogen | hydrogen | hydrogen | CONHCH₂-pyrrolidin-1-yl |
| (CMT-313) | hydrogen | hydrogen | hydrogen | CONHCH₂-piperadin-1-yl |
| (CMT-314) | hydrogen | hydrogen | hydrogen | CONHCH₂-morpholin-1-yl |
| (CMT-315) | hydrogen | hydrogen | hydrogen | CONHCH₂-piperazin-1-yl |

### General Formula (II)

Structures B through E represent the 4-dedimethylaminodoxycycline (CMT-8) derivatives 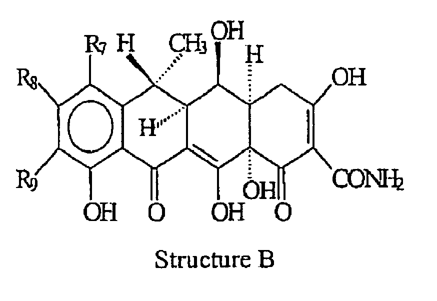 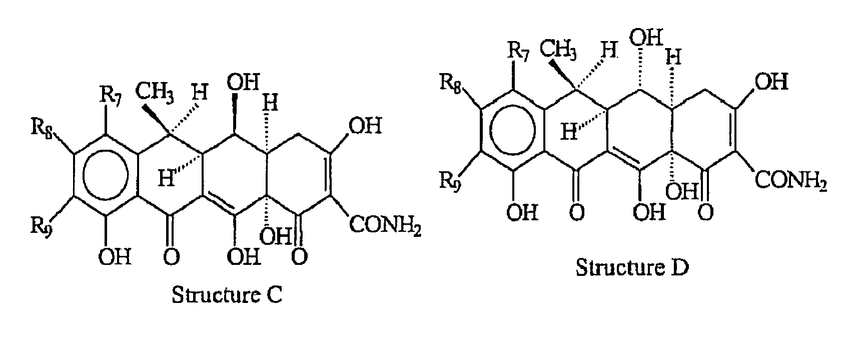 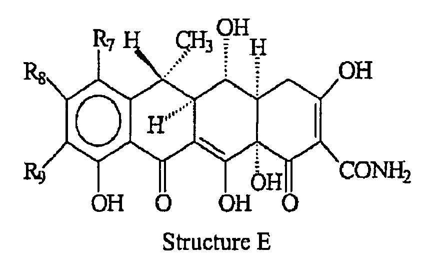 wherein R7, R8, and R9 taken together in each case, have the following meanings:

| | R7 | R8 | R9 |
|---|---|---|---|
| | | | |
| | azido | hydrogen | hydrogen |
| | dimethylamino | hydrogen | azido |
| | hydrogen | hydrogen | azido |
| | dimethylamino | hydrogen | amino |
| | acylamino | hydrogen | hydrogen |
| | hydrogen | hydrogen | acylamino |
| | amino | hydrogen | nitro |
| | hydrogen | hydrogen | (N,N-dimethyl)glycylamino |
| | amino | hydrogen | amino |
| | hydrogen | hydrogen | ethoxythiocarbonylthio |
| | dimethylamino | hydrogen | acylamino |
| | hydrogen | hydrogen | diazonium |
| | diazonium | hydrogen | hydrogen |
| | ethoxythiocarbonylthio | hydrogen | hydrogen |
| | dimethylamino | chloro | amino |
| | amino | chloro | amino |
| | acylamino | chloro | acylamino |
| | hydrogen | chloro | amino |
| | amino | chloro | hydrogen |
| | acylamino | chloro | hydrogen |
| | monoalkylamino | chloro | amino |
| | nitro | chloro | amino |
| (CMT-801) | hydrogen | hydrogen | acetamido |
| (CMT-802) | hydrogen | hydrogen | dimethylaminoacetamido |
| (CMT-803) | hydrogen | hydrogen | palmitamide |
| (CMT-804) | hydrogen | hydrogen | nitro |
| (CMT-805) | hydrogen | hydrogen | amino |
| (CMT-806) | hydrogen | hydrogen | dimethylamino |

| | R7 | R8 | R9 | R2 |
|---|---|---|---|---|
| (CMT-807) | hydrogen | hydrogen | hydrogen | CONHCH₂-pyrrolidin-1-yl |
| (CMT-808) | hydrogen | hydrogen | hydrogen | CONHCH₂-piperadin-1-yl |
| (CMT-809) | hydrogen | hydrogen | hydrogen | CONHCH₂-piperazine-1-yl |

### General Formula (III)

Structure F represents the 4-dedimethylaminominocycline (CMT-10) derivatives 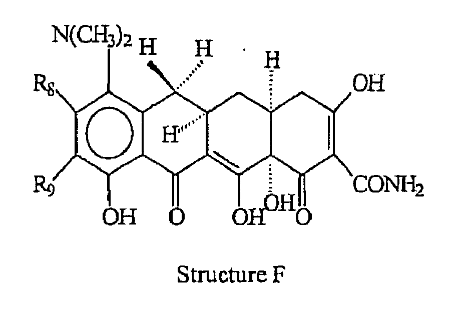 wherein R8 is hydrogen or halogen and R9 is selected from the group consisting of nitro (CMT-1002), (N,N-dimethyl)glycylamino, ethoxythiocarbonylthio. A compound related to structure F has a 7-trimethylammonium group instead of the 7-diemthylamino group, i.e. 7-trimethylammoniumsancycline (CMT-1001), and

### General Formula (IV)

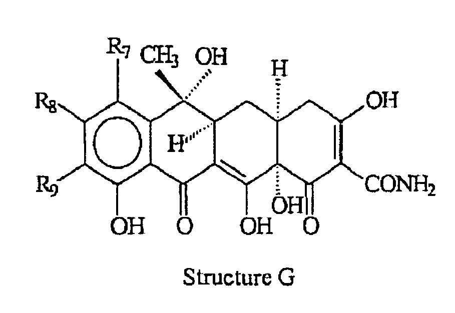

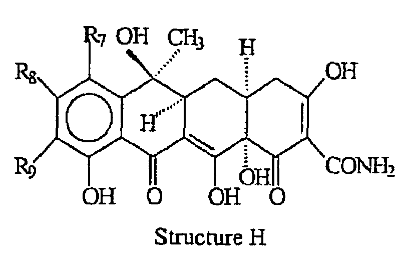

wherein R7, R8, and R9 taken together in each case, have the following "meanings:

| R7 | R8 | R9 |
|---|---|---|
| | | |
| amino | hydrogen | hydrogen |
| nitro | hydrogen | hydrogen |
| azido | hydrogen | hydrogen |
| dimethylamino | hydrogen | azido |
| hydrogen | hydrogen | amino |
| hydrogen | hydrogen | azido |
| hydrogen | hydrogen | nitro |
| bromo | hydrogen | hydrogen |
| dimethylamino | hydrogen | amino |
| acylamino | hydrogen | hydrogen |
| hydrogen | hydrogen | acylamino |
| amino | hydrogen | nitro |
| hydrogen | hydrogen | (N,N-dimethyl)glycylamino |
| amino | hydrogen | amino |
| diethylamino | hydrogen | hydrogen |
| hydrogen | hydrogen | ethoxythiocarbonylthio |
| dimethylamino | hydrogen | methylamino |
| dimethylamino | hydrogen | acylamino |
| dimethylamino | chloro | amino |
| amino | chloro | amino |
| acylamino | chloro | acylamino |
| hydrogen | chloro | amino |
| amino | chloro | hydrogen |
| acylamino | chloro | hydrogen |
| monoalkylamino | chloro | amino |
| nitro | chloro | amino |

Additional CMT's include, 4-dedimethylaminotetracycline (CMT-1), include tetracycline nitrile (CMT-2), 4-dedimethylaminochlorotetracycline (CMT-4), 4-dedimethylamino-4-hydroxytetracycline (CMT-6), 2a-dehydroxy-4-dedimethylaminotetracycline (CMT-7), and 1-deoxy-12a-dehydroxy-4-dedimethylaminotetracycline (CMT-9).

Non-antibacterial tetracycline compounds can be used in higher amounts than antibacterial tetracyclines, while avoiding the indiscriminate killing of bacteria, and the emergence of resistant bacteria. For example, 6-demethyl-6-deoxy-4-dedimethylaminotetracycline (CMT-3) may be administered in doses of about 10 to about 20mg/day which produces serum levels in humans of about 1µg/ml, or 40 to about 200mg/day, or in amounts that result in serum levels in humans of about 1.55µg/ml to about 10µg/ml.

The chemically modified tetracyclines can be made by methods known in the art. See, for example, Mitscher, L.A., The Chemistry of the Tetracycline Antibiotics, Marcel Dekker, New York (1978), Ch. 6, and U.S. Patents 4,704,383 and 5,532,227.

The invention also includes pharmaceutically acceptable salts of CMT-1002. The present invention embraces salts, including acid-addition and metal salts, of the 4-dedimethylaminotetraeycline compound CMT-1002. Such salts are formed by well known procedures. By "pharmaceutically acceptable" is meant those salt-forming acids and metals which do not substantially contribute to the toxicity of the compound.

Some examples of suitable salts include salts of mineral acids such as hydrochloric, hydriodic, hydrobromic, phosphoric, metaphosphoric, nitric and sulfuric acids, as well as salts of organic acids such as tartaric, acetic, citric, malic, benzoic, glycollic, gluconic, gulonic, succinic, arylsulfonic, e.g. p-toluenesulfonic acids, and the like.

After preparation, the compound of the present invention can be conveniently purified by standard methods known in the art. Some suitable examples include crystallization from a suitable solvent or partition-column chromatograph.

The preferred pharmaceutical composition for use in the method of the invention includes a combination of the tetracycline compound in a suitable pharmaceutical carrier (vehicle) or excipient as understood by practitioners in the art. Examples of carriers and excipients include starch, milk, sugar, certain types of clay, gelatin, stearic acid or salts thereof, magnesium or calcium stearate, talc, vegetable fats or oils, gums and glycols.

The tetracycline compound may be administered to mammals by sustained release, as is known in the art. Sustained release administration is a method of drug delivery to achieve a certain level of the drug over a particular period of time. The level typically is measured by serum concentration.

The tetracycline compound of the invention may be administered systemically. Systemic administration can be enteral or parenteral. Enteral administration is a preferred route of delivery of the tetracycline, and compositions including the tetracycline compound with appropriate diluents, carriers, and the like are readily formulated. Liquid or solid (e.g., tablets, gelatin capsules) formulations can be employed.

Administration can also be accomplished by a nebulizer or liquid mist. Nebulization is a preferred route of delivery of the tetracycline in situations where the respiratory system is particularly infected, for example, in the case of inhalation anthrax. By utilizing a nebulizer, the tetracycline is taken directly into the individuals respiratory system through inspiration.

Parenteral administration of the tetracycline compound of the invention (e.g., intravenous, intramuscular, subcutaneous injection) is also contemplated. Formulations using conventional diluents, carriers, etc. such as are known in the art can be employed to deliver the compound.

Alternatively, delivery of the tetracycline compound includes topical application. Topical administration is suitable in cutaneous infections such as, for example, cutaneous anthrax. Compositions deemed to be suited for such topical use include gels, salves, lotions, ointments and the like. Controlled release delivery of topical tetracyclines can be employed such as those currently used in dentistry such as ATRIDOX® (controlled release of topical doxycycline) and ARESTIN® (controlled release of topical minocycline).

The amount of tetracycline compound administered is any amount effective for reducing or inhibiting conditions associated with a bacterial exotoxin in the infected mammal. The actual preferred amounts of tetracycline compound in a specified case will vary according to the particular compositions formulated, the mode of application, and the particular subject being treated. The appropriate dose of the tetracycline compound can readily be determined by those skilled on the art.

The minimal amount of the tetracycline compound administered to a human is the lowest amount capable of providing effective treatment of the conditions. Effective treatment is a reduction or inhibition of the conditions, a reduction or inhibition of tissue destruction and/or prevention of death, of the mammal.

The maximal amount for a mammal is the highest amount that does not cause undesirable or intolerable side effects. Such doses can be readily determined by those skilled in the art. For example, CMTs can be systemically administered in a mammal in an amount of from about 0.05mg/kg/day to about 60mg/kg/day, and preferably from about 0.3mg/kg/day to about 18mg/kg/day. The practitioner is guided by skill and knowledge in the field, and the present invention includes, without limitation, dosages that are effective to achieve the desired antibacterial activity.

The appropriate dose of the tetracycline compound for topical administration can also be readily determined by those skilled in the art. For example, topical administration of CMTs in amounts of up to about 25% (w/w) in a vehicle can be administered without any toxicity in a human. Amounts from about 0.1% to about 10% are preferred.

The tetracycline of the present invention protect mammals at risk of acquiring a condition associated with the bacterial exotoxins calmodulin and/or metalloproteinase. Calmodulin exotoxin, otherwise known as adenylate cyclase toxin, catalyzes the conversion of ATP to cAMP. One example of a calmodulin exotoxin is the edema factor (EF) of anthrax (caused by *Bacillus anthracis*).

Metalloproteinase exotoxin is a peptide hydrolase which uses a metal, such as zinc, in the catalytic mechanism. For example, the lethal factor (LF) of anthrax is a zinc metalloproteinase exotoxin.

Many bacteria produce such exotoxins as part of their life cycle. For example, *Clostridium perfringens, Bordetella pertussis, Bacteriodes fragilis* and *Pseudomonas aeruginosa* are bacteria other than *Bacillus anthracis* that produce exotoxins.

The invention as claimed is limited to protecting against-, or treating, conditions associated with CI. perfringens, B. pertussis, B. fragilis or B. anthracis.

A mammal which can benefit from the methods of the present invention could be any mammal. Categories of mammals include, for example, humans, farm animals, domestic animals, laboratory animals, etc. Some examples of farm animals include cows, pigs, horses, goats, etc. Some examples of domestic animals include dogs, cats, etc. Some examples of laboratory animals include rats, mice, rabbits, guinea pigs, etc.

A mammal at risk of acquiring a condition associated with a bacteria that produces a calmodulin exotoxin or a metalloproteinase exotoxin, or both, includes mammals that have been, are suspected of having been, or are expected to be exposed to, or infected with, a bacteria that produces such exotoxins. Mammals that may have been exposed to a bacteria that produces a calmodulin exotoxin or a metalloproteinase exotoxin, or both, include, for example, military personnel, individuals that handle animal skins, individuals that live in endemic areas, health care professionals who may treat or have treated infected individuals, and individuals that have been in contact with, or in the vicinity of, an area that has tested positive for the presence of such bacteria.

The methods of the invention can also include administration of an antibiotic, such as ciprofloxacin or doxycycline, to an individual having a condition associated with bacteria that produce a calmodulin exotoxin, a metalloproteinase exotoxin, or both, along with the tetracycline. The antibiotic can be administered before, concurrently, or after the tetracycline is administered.

In one embodiment, the mammal may have received, or may be scheduled to receive, a vaccine against a bacteria that produces a calmodulin or metalloproteinase exotoxin, or both. The tetracycline of the present invention can be administered before, during or after a vaccine against a bacteria that produces a calmodulin or metalloproteinase exotoxin, or both is administered.

The tetracycline of the present invention provide a host with protection against conditions associated with the metalloproteinase and/or calmodulin bacterial exotoxins. Conditions associated with these bacterial exotoxins include, but are not limited to, the reactions that occur once a bacterium enters the host.

Examples of such conditions include hemolysis, inhibition of protein synthesis, flaccid paralysis, spastic paralysis, emesis, inflammation, fever, shock, localized erythematous reactions, tissue destruction, diarrhea and other conditions as are known in the art, including death.

### Clauses:

A method for protecting a mammal at risk of acquiring a condition associated with bacteria that produce a calmodulin exotoxin, a metalloproteinase exotoxin, or both, the method comprising administering to the mammal an effective amount of a non-antibacterial tetracycline, or a pharmaceutically acceptable salt thereof.
2. The method of clause 1 wherein the bacteria is selected from the group consisting of *Bacillus anthracis, Clostridium perfringens, Bordetella pertussis, Bacteriodes fragilis,* or *Pseudomonas aeruginosa.*
3. The method of clause 1 wherein the bacteria is *Bacillus anthracis*.
4. The method of clause 1 wherein the bacteria produces a calmodulin exotoxin.
5. The method of clause 1 wherein the bacteria produces a metalloproteinase exotoxin.
6. The method of clause 1 wherein the bacteria produces both a calmodulin exotoxin and a metalloproteinase exotoxin.
7. The method of clause 1 wherein the tetracycline is selected from the group consisting of CMT-1, CMT-2, CMT-4, CMT-6, CMT-7 or CMT-9, or pharmaceutically acceptable salts thereof.
8. The method of clause 1 wherein the tetracycline is selected from the group consisting of CMT-3, or its analogs, or pharmaceutically acceptable salts thereof.
9. The method according to clause 1 wherein the tetracycline is selected from the group consisting of CMT-8, or its analogs, or pharmaceutically acceptable salts thereof.
10. The method according to clause 1 wherein the tetracycline is selected from the group consisting of CMT-10, or its analogs, or pharmaceutically acceptable salts thereof.
11. A method for treating a mammal having a condition associated with bacteria that produce a calmodulin exotoxin, a metalloproteinase exotoxin, or both, the method comprising administering to the mammal an effective amount of a non-antibacterial tetracycline, or a pharmaceutically acceptable salt thereof.
12. The method of clause 11 wherein the bacteria is selected from the group consisting of *Bacillus anthracis, Clostridium perfringens, Bordetella pertussis, Bacteriodes fragilis,* or *Pseudomonas aeruginosa.*
13. The method of clause 11 wherein the bacteria is *Bacillus anthracis.*
14. The method of clause 11 wherein the bacteria produces a calmodulin exotoxin.
15. The method of clause 11 wherein the bacteria produces a metalloproteinase exotoxin.
16. The method of clause 11 wherein the bacteria produces both a calmodulin exotoxin and a metalloproteinase exotoxin.
17. The method of clause 11 wherein the tetracycline is selected from the group consisting of CMT-1, CMT-2, CMT-4, CMT-6, CMT-7 or CMT-9, or pharmaceutically acceptable salts thereof.
18. The method of clause 11 wherein the tetracycline is selected from the group consisting of CMT-3, or its analogs, or pharmaceutically acceptable salts thereof.
19. The method according to clause 11 wherein the tetracycline is selected from the group consisting of CMT-8, or its analogs, or pharmaceutically acceptable salts thereof.
20. The method according to clause 11 wherein the tetracycline is selected from the group consisting of CMT-10, or its analogs, or pharmaceutically acceptable salts thereof.
21. The method according to clause 11, further comprising an administering an antibiotic.
22. The method according to clause 21, wherein the antibiotic is ciprofloxacin.
23. The method according to clause 21, wherein the antibiotic is doxycycline.
24. A method for protecting a mammal at risk of acquiring a condition associated with bacteria that produce a calmodulin or a metalloproteinase exotoxin, or both, the method comprising administering to the mammal an effective, non-antibacterial amount of an antibacterial tetracycline, or a pharmaceutically acceptable salt thereof.
25. The method of clause 24, wherein the bacteria is selected from the group consisting of *Bacillus anthracis, Clostridium perfringens, Bordetella pertussis, Bacteriodes fragilis,* or *Pseudomonas aeruginosa.*
26. The method of clause 24, wherein the bacteria is *Bacillus anthracis.*
27. The method of clause 24, wherein the bacteria produces a calmodulin exotoxin.
28. The method of clause 24, wherein the bacteria produces a metalloproteinase exotoxin.
29. The method of clause 24, wherein the bacteria produces both a calmodulin exotoxin and a metalloproteinase exotoxin.
30. The method according to clause 24, wherein the tetracycline is selected from the group consisting of terramycin, aureomycin, doxycycline, minocycline, tetracycline, oxytetracycline, chlortetracycline, demeolocycline, lymecycline, or pharmaceutically acceptable salts thereof.
31. A method for treating a mammal having a condition associated with bacteria that produce a calmodulin or a metalloproteinase exotoxin, or both, the method comprising administering to the mammal an effective, non-antibacterial amount of an antibacterial tetracycline, or a pharmaceutically acceptable salt thereof.
32. The method of clause 31, wherein the bacteria is selected from the group consisting of *Bacillus anthracis, Clostridium perfringens, Bordetella pertussis, Bacteriodes fragilis,* or *Pseudomonas aeruginosa.*
33. The method of clause 31, wherein the bacteria is *Bacillus anthracis.*
34. The method of clause 31, wherein the bacteria produces a calmodulin exotoxin.
35. The method of clause 31, wherein the bacteria produces a metalloproteinase exotoxin.
36. The method of clause 31, wherein the bacteria produces both a calmodulin exotoxin and a metalloproteinase exotoxin.
37. The method according to clause 31, wherein the tetracycline is selected from the group consisting of terramycin, aureomycin, doxycycline, minocycline, tetracycline, oxytetracycline, chlortetracycline, demeclocycline, lymecycline, or pharmaceutically acceptable salts thereof.
38. The method according to clause 31, further comprising administering an antibiotic to the mammal.
39. The method according to clause 38, wherein the antibiotic is ciprofloxacin.
40. The method according to clause 38, wherein the antibiotic is doxycycline.
41. A method of protecting a mammal that has received or is scheduled to receive a vaccine against a bacteria that produces a calmodulin exotoxin, a metalloproteinase exotoxin, or both, the method comprising administering to the mammal an effective amount of a non-antibacterial tetracycline, or a pharmaceutically acceptable salt thereof.
42. The method of clause 41, wherein the bacteria is selected from the group consisting of *Bacillus anthracis, Clostridium perfringens, Bordetella pertussis, Bacteriodes fragilis,* or *Pseudomonas aeruginosa.*
43. The method of clause 41, wherein the bacteria is *Bacillus anthracis.*
44. The method of clause 41, wherein the bacteria produces a calmodulin exotoxin.
45. The method of clause 41, wherein the bacteria produces a metalloproteinase exotoxin.
46. The method of clause 41, wherein the bacteria produces both a calmodulin exotoxin and a metalloproteinase exotoxin.
47. The method of clause 41, wherein the tetracycline is selected from the group consisting of CMT-1, CMT-2, CMT-4, CMT-6, CMT-7 or CMT-9, or pharmaceutically acceptable salts thereof
48. The method of clause 41, wherein the tetracycline is selected from the group consisting of CMT-3, or its analogs, or pharmaceutically acceptable salts thereof.
49. The method according to clause 41, wherein the tetracycline is selected from the group consisting of CMT-8, or its analogs, or pharmaceutically acceptable salts thereof.
50. The method according to clause 41, wherein the tetracycline is selected from the group consisting of CMT-10, or its analogs, or pharmaceutically acceptable salts thereof.
51. The method according to clause 41, wherein the tetracycline is administered before the vaccine is administered.
52. The method according to clause 41, wherein the tetracycline is administered at the same time that the vaccine is administered.
53. The method according to clause 41, wherein the tetracycline is administered after the vaccine is administered.
54. A method of protecting a mammal that has received or is scheduled to receive a vaccine against a bacteria that produces a calmodulin exotoxin, a metalloproteinase exotoxin, or both, the method comprising administering to the mammal an effective, non-antibacterial amount of an antibacterial tetracycline, or a pharmaceutically acceptable salt thereof.
55. The method of clause 54, wherein the bacteria is selected from the group consisting of *Bacillus anthracis, Clostridium perfringens, Bordetella pertussis, Bacteriodes fragilis,* or *Pseudomonas aeruginosa.*
56. The method of clause 54, wherein the bacteria is *Bacillus anthracis.*
57. The method of clause 54, wherein the bacteria produces a calmodulin exotoxin.
58. The method of clause 54, wherein the bacteria produces a metalloproteinase exotoxin.
59. The method of clause 54, wherein the bacteria produces both a calmodulin exotoxin and a metalloproteinase exotoxin.
60. The method according to clause 54, wherein the tetracycline is selected from the group consisting of terramycin, aureomycin, doxycycline, minocycline, tetracycline, oxytetracycline, chlortetracycline, demeclocycline, lymecycline, or pharmaceutically acceptable salts thereof.
61. The method according to clause 54, wherein the tetracycline is administered before the vaccine is administered.
62. The method according to clause 54, wherein the tetracycline is administered at the same time that the vaccine is administered.
63. The method according to clause 54, wherein the tetracycline is administered after the vaccine is administered.

The above defined "clauses" do not form part of the invention as claimed.

## Claims

1. CMT-1002 or a pharmaceutically acceptable salt thereof, for use in protecting a mammal at risk of acquiring a condition associated with bacteria selected from the group consisting of *Bacillus anthracis, Clostridium perfringens, Bordetella pertussis,* and *Bacteriodes fragilis,* wherein an effective amount of said CMT-1002 or a pharmaceutically acceptable salt thereof is to be administered to the mammal.

2. CMT-1002 or a pharmaceutically acceptable salt thereof, for use in treating a mammal having a condition associated with bacteria selected from the group consisting of *Bacillus anthracis, Clostridium perfringens, Bordetella pertussis,* and *Bacteriodes fragilis,* wherein an effective amount of said CMT-1002 or a pharmaceutically acceptable salt thereof is to be administered to the mammal.

3. CMT-1002 or a pharmaceutically acceptable salt thereof according to claim 2, wherein further an antibiotic is to be administered.

4. CMT-1002 or a pharmaceutically acceptable salt thereof according to claim 3, wherein the antibiotic is ciprofloxacin.

5. CMT-1002 or a pharmaceutically acceptable salt thereof according to claim 3, wherein the antibiotic is doxycycline.

6. CMT-1002 or a pharmaceutically acceptable salt thereof, for use in protecting a mammal that has received or is scheduled to receive a vaccine against bacteria selected from the group consisting of *Bacillus anthracis, Clostridium perfringens, Bordetella pertussis,* and *Bacteriodes fragilis,* wherein an effective amount of said CMT-1002 or a pharmaceutically acceptable salt thereof is to be administered to the mammal.

7. CMT-1002 or a pharmaceutically acceptable salt thereof for use according to any of the previous claims, wherein the bacteria is *Bacillus anthracis.*

8. CMT-1002 or a pharmaceutically acceptable salt thereof for use according to claim 6 or 7, wherein the CMT-1002 or the pharmaceutically acceptable salt thereof is to be administered before the vaccine is administered.

9. CMT-1002 or a pharmaceutically acceptable salt thereof for use according to any of the claims 6-8, wherein the CMT-1002 or the pharmaceutically acceptable salt thereof is administered at the same time that the vaccine is administered.

10. CMT-1002 or a pharmaceutically acceptable salt thereof for use according to any of the claims 6-8, wherein the CMT-1002 or the pharmaceutically acceptable salt thereof is administered after the vaccine is administered.

11. CMT-1002 or a pharmaceutically acceptable salt thereof for use according to any of the previous claims, wherein CMT-1002 or the pharmaceutically acceptable salt thereof is to be administered by sustained release.

12. CMT-1002 or a pharmaceutically acceptable salt thereof for use according to any of the previous claims, wherein CMT-1002 or the pharmaceutically acceptable salt thereof is to be administered systemically.

13. CMT-1002 or a pharmaceutically acceptable salt thereof for use according to claim 12, wherein CMT-1002 or the pharmaceutically acceptable salt thereof is to be administered enterally.

14. CMT-1002 or a pharmaceutically acceptable salt thereof for use according to claim 12, wherein CMT-1002 or the pharmaceutically acceptable salt thereof is to be administered in a dose of from about 0.05 mg/kg/day to about 60 mg/kg/day.

## Patentansprüche

1. CMT-1002 oder ein pharmazeutisch akzeptables Salz davon, zur Verwendung für den Schutz eines Säugers, der Gefahr läuft, einen Zustand in Zusammenhang mit Bakterien, ausgewählt aus der Gruppe bestehend aus *Bacillus anthracis, Clostridium perfringens, Bordetella pertussis* und *Bacteriodes fragilis* zu erleiden, wobei eine wirksame Menge des CMT-1002 oder eines pharmazeutisch akzeptablen Salzes davon dem Säuger zu verabreichen ist.

2. CMT-1002 oder ein pharmazeutisch akzeptables Salz davon, zur Verwendung bei der Behandlung eines Säugers mit einem Zustand in Zusammenhang mit Bakterien, ausgewählt aus der Gruppe bestehend aus *Bacillus anthracis, Clostridium perfringens, Bordetella pertussis* und *Bacteriodes fragilis,* wobei eine wirksame Menge des CMT-1002 oder eines pharmazeutisch akzeptablen Salzes davon dem Säuger zu verabreichen ist.

3. CMT-1002 oder ein pharmazeutisch akzeptables Salz davon nach Anspruch 2, wobei ferner ein Antibiotikum zu verabreichen ist.

4. CMT-1002 oder ein pharmazeutisch akzeptables Salz davon nach Anspruch 3, wobei das Antibiotikum Ciprofloxacin ist.

5. CMT-1002 oder ein pharmazeutisch akzeptables Salz davon nach Anspruch 3, wobei das Antibiotikum Doxycyclin ist.

6. CMT-1002 oder ein pharmazeutisch akzeptables Salz davon, zur Verwendung für den Schutz eines Säugers, bei dem eine Impfung gegen Bakterien, ausgewählt aus der Gruppe bestehend aus *Bacillus anthracis, Clostridium perfringens, Bordetella pertussis* und *Bacteriodes fragilis* vorgenommen wurde oder geplant ist, wobei eine wirksame Menge des CMT-1002 oder eines pharmazeutisch akzeptablen Salzes davon dem Säuger zu verabreichen ist.

7. CMT-1002 oder ein pharmazeutisch akzeptables Salz davon nach einem der vorhergehenden Ansprüche, wobei das Bakterium *Bacillus anthracis* ist.

8. CMT-1002 oder ein pharmazeutisch akzeptables Salz davon nach Anspruch 6 oder 7, wobei das CMT-1002 oder das pharmazeutisch akzeptable Salz davon zu verabreichen ist bevor der Impfstoff verabreicht wird.

9. CMT-1002 oder ein pharmazeutisch akzeptables Salz davon nach einem der Ansprüche 6-8, wobei das CMT-1002 oder das pharmazeutisch akzeptable Salz davon zum gleichen Zeitpunkt zu verabreichen ist an dem der Impfstoff verabreicht wird.

10. CMT-1002 oder ein pharmazeutisch akzeptables Salz davon zur Verwendung nach einem der Ansprüche 6-8, wobei das CMT-1002 oder das pharmazeutisch akzeptable Salz davon zu verabreichen ist nachdem der Impfstoff verabreicht wird.

11. CMT-1002 oder ein pharmazeutisch akzeptables Salz davon nach einem der vorhergehenden Ansprüche, wobei das CMT-1002 oder das pharmazeutisch akzeptable Salz davon durch anhaltende Freisetzung zu verabreichen ist.

12. CMT-1002 oder ein pharmazeutisch akzeptables Salz davon nach einem der vorhergehenden Ansprüche, wobei das CMT-1002 oder das pharmazeutisch akzeptable Salz davon systematisch zu verabreichen ist.

13. CMT-1002 oder ein pharmazeutisch akzeptables Salz davon nach Anspruch 12, wobei das CMT-1002 oder das pharmazeutisch akzeptable Salz davon enteral zu verabreichen ist.

14. CMT-1002 oder ein pharmazeutisch akzeptables Salz davon nach Anspruch 12, wobei das CMT-1002 oder das pharmazeutisch akzeptable Salz davon in einer Dosis von ungefähr 0,05 mg/kg/Tag bis ungefähr 60 mg/kg/Tag zu verabreichen ist.

## Revendications

1. CMT-1002 ou sel pharmaceutiquement acceptable de celui-ci, destiné à être utilisé dans la protection d'un mammifère risquant de contracter une pathologie associée à des bactéries choisies dans le groupe consistant en *Baci*//*us anthracis, Clostridium perfringens, Bordetella pertussis* et *Bacteriodes fragilis,* où une quantité efficace dudit CMT-1002 ou d'un sel pharmaceutiquement acceptable de celui-ci est destinée à être administrée au mammifère.

2. CMT-1002 ou sel pharmaceutiquement acceptable de celui-ci destiné à être utilisé dans le traitement d'un mammifère ayant une pathologie associée à des bactéries choisies dans le groupe consistant en *Baci*//*us anthracis, Clostridium perfringens, Bordetella pertussis* et *Bacteriodes fragilis,* où une quantité efficace dudit CMT-1002 ou d'un sel pharmaceutiquement acceptable de celui-ci est destinée à être administrée au mammifère.

3. CMT-1002 ou sel pharmaceutiquement acceptable de celui-ci selon la revendication 2, où un antibiotique est destinée à être en outre administré.

4. CMT-1002 ou sel pharmaceutiquement acceptable de celui-ci selon la revendication 3, où l'antibiotique est la ciprofloxacine.

5. CMT-1002 ou sel pharmaceutiquement acceptable de celui-ci selon la revendication 3, où l'antibiotique est la doxycycline.

6. CMT-1002 ou sel pharmaceutiquement acceptable de celui-ci destiné à être utilisé dans la protection d'un mammifère ayant reçu ou devant recevoir un vaccin contre des bactéries choisies dans le groupe consistant en *Baci*//*us anthracis, Clostridium perfringens, Bordetella pertussis* et *Bacteriodes fragilis,* où une quantité efficace dudit CMT-1002 ou d'un sel pharmaceutiquement acceptable de celui-ci est destinée à être administrée au mammifère.

7. CMT-1002 ou sel pharmaceutiquement acceptable de celui-ci destiné à être utilisé selon l'une quelconque des revendications précédentes, où les bactéries sont *Baci*//*us anthracis.*

8. CMT-1002 ou sel pharmaceutiquement acceptable de celui-ci destiné à être utilisé selon la revendication 6 ou 7, où le CMT-1002 ou le sel pharmaceutiquement acceptable de celui-ci est destiné à être administré avant l'administration du vaccin.

9. CMT-1002 ou sel pharmaceutiquement acceptable de celui-ci destiné à être utilisé selon l'une quelconque des revendications 6-8, où le CMT-1002 ou le sel pharmaceutiquement acceptable de celui-ci est destiné à être administré au moment de l'administration du vaccin.

10. CMT-1002 ou sel pharmaceutiquement acceptable de celui-ci destiné à être utilisé selon l'une quelconque des revendications 6-8, où le CMT-1002 ou le sel pharmaceutiquement acceptable de celui-ci est destiné à être administré après l'administration du vaccin.

11. CMT-1002 ou sel pharmaceutiquement acceptable de celui-ci destiné à être utilisé selon l'une quelconque des revendications précédentes, où CMT-1002 ou le sel pharmaceutiquement acceptable de celui-ci est destiné à être administré par libération prolongée.

12. CMT-1002 ou sel pharmaceutiquement acceptable de celui-ci destiné à être utilisé selon l'une quelconque des revendications précédentes, où CMT-1002 ou le sel pharmaceutiquement acceptable de celui-ci est destiné à être administré de manière systémique.

13. CMT-1002 ou sel pharmaceutiquement acceptable de celui-ci destiné à être utilisé selon la revendication 12, où CMT-1002 ou le sel pharmaceutiquement acceptable de celui-ci est destiné à être administré de manière entérale.

14. CMT-1002 ou sel pharmaceutiquement acceptable de celui-ci destiné à être utilisé selon la revendication 12, où CMT-1002 ou le sel pharmaceutiquement acceptable de celui-ci est destiné à être administré en une dose d'environ 0,05 mg/kg/jour à environ 60 mg/kg/jour.
